# EUROPÄISCHE PATENTSCHRIFT

(11) **EP 0 613 365 B1**
(45) Veröffentlichungstag und Bekanntmachung des Hinweises auf die Patenterteilung: **02.08.1995**
(21) Anmeldenummer: 92922935.9
(22) Anmeldetag: 09.11.1992
(51) Int. Cl.: A61K 7/13

(54) **HAARTÖNUNGSMITTEL**
HAIR-TINTING AGENTS
AGENTS DE COLORATION DES CHEVEUX

(30) Priorität: 18.11.1991 DE 4137954
(43) Veröffentlichungstag der Anmeldung: 07.09.1994
(73) Patentinhaber: Henkel Kommanditgesellschaft auf Aktien, 40191 Düsseldorf (DE)
(72) Erfinder: MATZIK, Iduna, D-4006 Erkrath 2 (DE); BORGERDING, Mechthild, D-4018 Langenfeld (DE); HOLLENBERG, Detlef, D-4006 Erkrath 2 (DE)
(86) Internationale Anmeldenummer: EP9202571
(87) Internationale Veröffentlichungsnummer: WO9309758

(56) Entgegenhaltungen:
- WO-A-90/01922
- DE-A- 1 916 139
- GB-A- 2 093 868
- GB-A- 2 181 750

## Beschreibung

Die Erfindung betrifft Haartönungsmittel mit einem Gehalt an natürlichen und synthetischen Farbstoffen in einem kosmetischen Träger.

Für das Färben von Humanhaar werden überwiegend synthetische Farbstoffe verwendet, von welchen die Oxidationsfärbemittel wegen ihrer höheren Intensität und Echtheit die größere Bedeutung für Permanentfärbungen haben. Für semipermanente Färbungen und Tönungen werden auch direktziehende Farbstoffe verwendet.

Um toxikologische Risiken zu vermeiden, ist in jüngster Zeit ein Bestreben festzustellen, Naturfarbstoffe anstelle von syntehtischen Farbstoffen zu verwenden. Wegen ihres schwachen Aufziehvermögens weisen viele der bekannten natürlichen Farbstoffe nur geringe Färbeeigenschaften auf.

Es wurde auch schon versucht, Naturfarbstoffe und synthetische Färbemittel kombiniert einzusetzen. Z. B. wurde in WO 90/01922 vorgeschlagen, in einem zweistufigen Färbeprozeß zuerst einen synthetischen Direktfarbstoff und dann einen Naturfarbstoff aufzubringen.

Schließlich hat die Anmelderin gefunden, daß bei der Verwendung von 4,6-Dihydroxyindolin als Oxidationsfarbstoffvorprodukt in Oxidationshaarfärbemitteln durch das "in situ" gebildete 5,6-Dihydroxyindol sehr naturnahe Haarfärbungen erzielt werden.

Nunmehr wurde festgestellt, daß sich 5,6-Dihydroxyindolin auch sehr gut dazu eignet, Färbemittel auf Basis von Naturfarbstoffen in ihrer Nuance zu modifizieren um intensivere Haaranfärbungen mit höheren Echtheitseigenschaften zu erhalten.

Gegenstand der Erfindung sind daher Haartönungsmittel mit einem Gehalt an natürlichen und synthetischen Farbstoffen in einem kosmetischen Träger, die als Färbemittel eine Kombination aus
(A) wenigstens einem wasserlöslichen Naturfarbstoff und
(B) wenigstens einem Indolin der Formel I 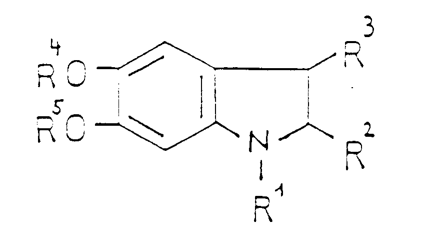 worin R¹, R², R³, R⁴ und R⁵ unabhängig voneinander Wasserstoff oder Alkylgruppen mit 1 - 4 C-Atomen oder R⁴ und R⁵ gemeinsam mit den Sauerstoffatomen, an die sie gebunden sind, eine Alkylendioxygruppe mit 1 - 4 C-Atomen darstellen, oder dessen Salz enthalten.

Solche erfindungsgemäßen Haartönungsmittel werden ohne Zusatz von Oxidationsmitteln auf das Haar aufgebracht. Die gewünschte Färbung wird durch bloße Trocknung der angefärbten Haare an der Luft erzielt.

Als Naturfarbstoffe eignen sich dabei alle in Pflanzen, Tieren und Mikroorganismen enthaltenen und aus diesen gewinnbaren Farbstoffe, insbesondere solche, die bereits zur Färbung von Keratinfasern oder von Haaren verwendet worden sind. Eine Übersicht über natürliche Farbstoffe ist aus Ullmanns Encyclopädie der technischen Chemie, 4. Auflage (19), Band 11, Seite 100 - 134 zu entnehmen. Von den dort angegebenen Farbstoffen sind besonders geeignet
- β-Carotin (aus Karotten),
- Carmin (ein Aluminium-Komplexsalz der aus Cochenille gewinnbaren Carminsäure),
- Lawson (2-Hydroxy-1,4-naphthochinon aus Lawsonia alba (Henna)),
- Santaline (aus Sandelholz),
- Bixin (aus Bixa orellana),
- Quercetin (aus der Färbereiche (Quercus tinctoria)),
- Hämatoxylin (aus Extrakten des Blauholzbaumes).

Von den Indolinen der Formel I eignet sich bevorzugt das 5,6-Dihydroxyindolin, also das Indolinderivat der Formel I, worin R¹, R², R³, R⁴ und R⁵ Wasserstoff ist, da dieses durch Oxidation an der Luft 5,6-Dihydroxyindol und weiterhin Melanin-Farstoffe bildet. Es ist aber bekannt, daß auch Derivate des 5,6-Dihydroxyindols zu melanin-ähnlichen Farbstoffen oxydiert werden. Entsprechend eignen sich auch die alkylsubstituierten Indoline der Formel I, bevorzugt jene, bei welchen eine der Gruppen R¹, R² und R³ eine Methylgruppe ist und die übrigen Wasserstoff sind, als synthetische Indolin-Farbstoffe (B) in der erfindungsgemäßen Farbstoffkombination.

Durch die erfindungsgemäße Kombination werden die Nuancen der Naturfarbstoffe in unerwarteter Weise modifiziert, wobei neue Farbtönungen erhalten werden, die nicht durch einfache Farbaddition erzielbar sind:
So führt rotes Carmin in Kombination mit braunfärbendem Indolin zu einem blaugrauen Farbton auf blondem Haar. Graues Haar mit 20 % Braun-Anteil wird dunkelblau bis schwarz gefärbt.

Lawson (Henna) ergibt in Kombination mit dem braunfärbenden 5,6-Dihydroxyindolin auf hellblondem Haar einen gedeckten Kupferton, auf grauem Haar eine warme, rotbraune Nuance.

Gelbes Quercetin färbt in Kombination mit 5,6-Dihydroxyindolin blondes Haar dunkel altrosa, graues Haar jedoch schwarz-braun.

Je nach der gewünschten Nuance werden die Naturfarbstoffe und das Indolin der Formel I in Mengenverhältnissen von 9 : 1 bis 1 : 9 eingesetzt. Bevorzugt werden beide Farbstoffe in etwa gleichen Mengen verwendet.

Es können auch mehrere unterschiedliche Naturfarbstoffe oder mehrere Indoline der Formel I gleichzeitig in einem erfindungsgemäßen Haartönungsmittel enthalten sein.

Zur Herstellung der erfindungsgemäßen Haartönungsmittel werden die Naturfarbstoffe und das Indolin in einem geeigneten kosmetischen Träger eingearbeitet.

Solche Träger sind z. B. Cremes, Emulsionen, Gele oder auch tensidhaltige, schäumende Lösungen (Shampoos), Schaumaerosole oder sonstige Zubereitungen, die für die Anwendung auf dem Haar geeignet sind. Sie enthalten Konfektionierung- und Färbehilfsmittel, welche die Stabilität der Zubereitungen erhöhen und das Ergebnis der Färbung verbessern. Solche Zusätze sind in erster Linie oberflächenaktive Stoffe, z. B.
- Seifen, insbesondere die Alkali- oder Alkanolaminseifen von linearen C₁₂-C₁₈-Fettsäuren, insbesondere von Ölsäure,
- anionische Tenside, z. B. Fettalkoholsulfate und Fettalkoholpolyglycolethersulfate, Alkansulfonate, alpha-Olefinsulfonate oder Ölsäuresulfonate, bevorzugt in Form der Alkali-, Ammonium- oder oder Alkanolammoniumsalze,
- kationische Tenside, z. B. Alkyl(C₁₂-C₁₈)-trimethyl-ammoniumchloride, Alkyl(C₁₂-C₁₈)-dimethyl-benzyl-ammoniumsalze, Cetyl-pyridiniumchlorid, 2-Hydroxydodecyl-hydroxyethyl-dimethylammoniumchlorid,
- zwitterionische Tenside, wie z. B. Alkyl-(C₁₂-C₁₈)-dimethyl-ammonium-glycinat, Kokosacylaminopropyl-dimethyl-ammonium-glycinat, oder Imidazoliniumbetaine,
- amphotere Tenside, wie z. B. N-Dodecylaminoessigsäure, N-Cetyl-aminopropionsäure, gamma-Laurylamino-buttersäure und
- nichtionische Tenside, insbesondere Anlagerungsprodukte von 5 bis 30 Mol Ethylenoxid an Fettalkohole, an Alkylphenole, an Fettsäuren, an Fettsäurealkanolamide, an Fettsäurepartialglyceride, an Fettsäure-sorbitanpartialester oder an Fettsäure-methylglucosid-partialester, ferner Alkylglucoside, Aminoxide und Fettsäure-polyglycerinester.

Weitere Konfektionierungshilfsmittel sind die
- wasserlöslichen, verdickenden Polymeren (Hydrokolloide), z. B. Celluloseether, wie Carboxymethylcellulose, Hydroxyethylcellulose, Methylcellulose, Methyl-hydroxypropylcellulose, Stärke und Stärkeether, Pflanzengumme, Guar-Gum, Agar-Agar, Alginate, Xanthan-Gum oder syntehtische wasserlösliche Polymere,
- Puffersubstanzen, z. B. Ammoniumchlorid und Ammoniumsulfat,
- Komplexbildner, z. B. 1-Hydroxyethan-1,1-diphosphonsäure, Nitrilotriessigsäure oder Ethylendiamintetraessigsäure oder deren Salze,
- haarkosmetische Hilfsmittel, z. B. wasserlösliche kationische Polymere, Proteinderivate, Glucose, D-Panthenol, Cholesterin, Vitamine oder Pflanzenextrakte,
- Egalisierhilfsmittel, z. B. Urazol, Hexahydropyrimidin-2-on, Imidazol, 1,2,4-Triazol oder Jodide, z. B. Natrium- oder Kaliumjodid.

Die Anwendung der erfindungsgemäßen Haarfärbemittel kann im schwach sauren, neutralen oder schwach alkalischen pH-Bereich erfolgen.

Als schwach basische Träger für die Färbezubereitung eignen sich bevorzugt Gele oder Öl-in-Wasser-Emulsionen. Geeignete Gele enthalten als oberflächenaktive Stoffe 1 - 20 Gew.-% Seife, bevorzugt Ammoniumoleat, bevorzugt zusätzlich 1 - 10 Gew.-% eines nichtionischen Emulgators und 5 - 20 Gew.-% eines Fettalkohols mit 12 - 22 C-Atomen als Fettkomponente. Geeignete Öl-in-Wasser-Emulsionen enthalten 1 - 25 Gew.-% einer Fettkomponente, bevorzugt eines Fettalkohols mit 12 - 22 C-Atomen, und 0,5 - 30 Gew.-% eines Emulgiermittels, bevorzugt 1 - 20 Gew.-% eines anionischen, nichtionischen, zwitterionischen oder ampholytischen Tensids.

Eine bevorzugte Ausführungsform der Erfindung besteht darin, daß als Träger eine wäßrige Emulsion von 2 - 20 Gew.-% eines oder mehrerer Fettalkohole mit 12 - 22 C-Atomen und als Farbstoffe 0,1 - 5 Gew.-% eines Naturfarbstoffs und 0,1 - 5 Gew.-% 5,6-Dihydroxyindolin, jeweils bezogen auf das gesamte Mittel, enthalten sind. Der pH-Wert dieser bevorzugten Haartönungsmittel wird auf Werte zwischen 8,5 und 10,5 eingestellt.

Als Träger eignen sich aber auch wasserfreie pulverförmige oder granulierte Zubereitungen die sich durch Auflösen oder Dispergieren in Wasser zu streichfähigen Gelen, Emulsionen oder Dispersionen verarbeiten lassen.

Die Anwendung der erfindungsgemäßen Haartönungsmittel erfolgt bevorzugt in der Weise, daß man die Zubereitung von
0,1 - 5 Gew.-% des Naturfarbstoffs und
0,1 - 5 Gew.-% des Indolins der Formel I
in einem emulsionsförmigen oder gelförmigen wäßrigen Träger auf das Haar aufbringt, nach einer Einwirkungszeit von 10 - 40 Minuten überschüssiges Färbemittel mit Wasser abspült und das Haar an der Luft trocknet. Während dieses Vorgangs bildet sich die Nuance durch Aufzug der Farbstoffe auf die Faser und oxidative Entwicklung der Melanin-Farbstoffe unter dem Einfluß des Luftsauerstoffes aus.

Die folgenden Beispiele sollen den Erfindungsgegenstand näher erläutern.

### Beispiel

Es wurde eine Färbecreme-Emulsion der folgenden Zusammensetzung hergestellt:

| | |
|---|---|
| Kokosfettalkohol C₁₂-C₁₈ | 2,0 |
| hydr. Talgfettalkohol | 6,0 |
| hydr. Talgfettalkohol-polyglycolether (25 EO) | 2,0 |
| Wasser | 60,0 |
| 5,6-Dihydroxyindolin-hydrobromid | 1,0 |
| Naturfarbstoff | 1,0 |
| konz. Ammoniak-Lösung bis pH = 10 | - |
| Wasser | ad 100,0 |

Es wurde zunächst eine Emulsion der Fettalkohole hergestellt. In diese wurde eine wäßrige Lösung der Farbstoffe eingearbeitet und mit Ammoniak der pH-Wert auf 10 gestellt, dann wurde mit Wasser auf 100 g ergänzt.

Als Naturfarbstoffe wurden nacheinander die Stoffe
Carmin
Lawson und
Quercetin
eingearbeitet.

Die Färbecremes wurden auf ca. 5 cm lange Strähnen standardis erten, zu 90 % ergrauten, aber nicht besonders vorbehandelten n-schenhaars aufgetragen und dort 30 Minuten bei 25 °C belassen.

In gleicher Weise wurde auch eine blonde Haarsträhne behandelt.

Nach Beendigung des Färbeprozesses wurden die Haarsträhnen mit Wasser ausgespült und mit Luft von 60 °C getrocknet.

Mit den geprüften Naturfarbstoffen wurden die folgenden Haartönungen erhalten:

| Naturfarstoff | Nuance | |
|---|---|---|
| | der grauen Strähne | der blonden Strähne |
| Carmin | stahlblau bis schwarz | blaugrau |
| Lawson | warmes rotbraun | gedeckt kupferfarbig |
| Quercetin | schwarz-braun | dunkles alt-rosa |

Intensität und Echtheit, insbesondere die Waschechtheit, der Färbungen ist erheblich höher als bei Verwendung der Naturfarbstoffe allein.

## Patentansprüche

1. Haartönungsmittel mit einem Gehalt an natürlichen und synthetischen Farbstoffen in einem kosmetischen Träger, dadurch gekennzeichnet, daß als Farbstoffe eine Kombination von
(A) wenigstens einem Naturfarbstoff und
(B) wenigstens einem Indolin der Formel I 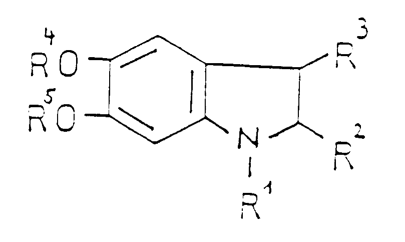 worin R¹, R², R³, R⁴ und R⁵ unabhängig voneinander Wasserstoff oder Alkylgruppen mit 1 - 4 C-Atomen oder R⁴ und R⁵ gemeinsam mit den Sauerstoffatomen, an die sie gebunden sind, eine Alkylendioxygruppe mit 1 - 4 C-Atomen darstellen, oder dessen Salz enthält.

2. Haartönungsmittel nach Anspruch 1, dadurch gekennzeichnet, daß der Naturfarbstoff ausgewählt ist aus β-Carotin, Carmin, Lawson, Santalin, Bixin, Quercetin und Hämatoxylin.

3. Haartönungsmittel nach Anspruch 1 oder 2, dadurch gekennzeichnet, daß als Träger eine wäßrige Emulsion von 2 - 20 Gew.-% eines oder mehrerer Fettalkohole mit 12 - 22 C-Atomen, bezogen auf das gesamte Mittel und als Farbstoffe 0,1 - 5 Gew.-% eines Naturfarbstoffs und 0,1 - 5 Gew.-% 5,6-Dihydroxyindolin, bezogen auf das gesamte Mittel, enthalten sind.

4. Verfahren zur Tönung von Haaren, dadurch gekennzeichnet, daß man eine Zubereitung von
(A) 0,1 - 5 Gew.-% eines Naturfarbstoffs und
(B) 0,1 - 5 Gew.-% eines Indolins der Formel I gemäß Patentanspruch 1
in einem emulsionsförmigen oder gelförmigen wäßrigen Träger auf das Haar aufbringt, nach einer Einwirkungszeit von 10 - 40 Minuten überschüssiges Färbemittel mit Wasser abspült und das Haar an der Luft trocknet.

## Claims

1. Hair tinting formulations containing natural and synthetic dyes in a cosmetic carrier, characterized in that they contain as dyes a combination of
(A) at least one natural dye and
(B) at least one indoline corresponding to formula I: 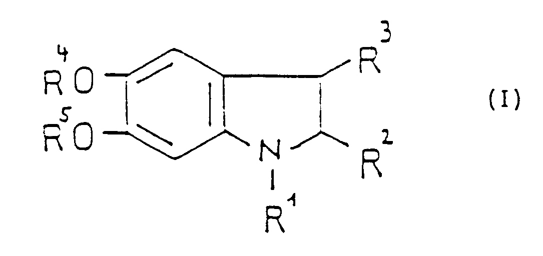 in which R¹, R², R³, R⁴ and R⁵ independently of one another represent hydrogen or alkyl groups containing 1 to 4 carbon atoms or R⁴ and R⁵ together with the oxygen atoms to which they are attached represent an alkylenedioxy group containing 1 to 4 carbon atoms,
or a salt thereof.

2. Hair-tinting formulations as claimed in claim 1, characterized in that the natural dye is selected from β-carotene, carmine, lawson, santalin, bixin, quercetin and haematoxylin.

3. Hair-tinting formulations as claimed in claim 1 or 2, characterized in that they contain an aqueous emulsion of 2 to 20% by weight of one or more C₁₂₋₂₂ fatty alcohols, based on the formulation as a whole, as the carrier and 0.1 to 5% by weight of a natural dye and 0.1 to 5% by weight of 5,6-dihydroxyindoline, based on the formulation as a whole, as the dyes.

4. A process for tinting hair, characterized in that a preparation of
(A) 0.1 to 5% by weight of a natural dye and
(B) 0.1 to 5% by weight of an indoline corresponding to formula I in claim 1
in an emulsion-form or gel-form agueous carrier is applied to the hair, excess dye is rinsed off with water after a contact time of 10 to 40 minutes and the hair is dried in air.

## Revendications

1. Agents de coloration des cheveux ayant une teneur en matières colorantes synthétiques dans un support cosmétique, caractérisés en ce que comme matières tinctoriales ils contiennent une combinaison de :
A) au moins un produit colorant naturel soluble dans l'eau et,
B) au moins un indoline de formule I 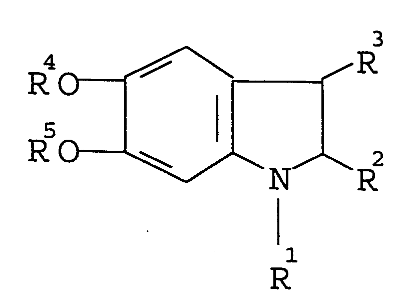 dans laquelle,
R¹, R², R³ R⁴ et R⁵ représentent indépendamment l'un de l'autre de l'hydrogène ou des groupes alkyle ayant 1-4 atomes de C ou R⁴ et R⁵ représentent ensemble avec les atomes d'oxygène, auxquels ils sont liés, un groupe alkylènedioxyde ayant 1-4 atomes de C, ou son sel.

2. Agent de coloration des cheveux selon la revendication 1, caractérisé en ce que la substance colorante naturelle est choisie à partir de caratine β, carmin, lawson, santaline, lixine, quercétine et hématoxyline.

3. Agent de coloration des cheveux selon les revendications 1 ou 2, caractérisé en ce que comme porteur est contenue une émulsion aqueuse de 2-20 % en poids d'un ou plusieurs alcools gras ayant 12-22 atomes de C, par rapport à l'agent total et comme substances colorantes 0,1-5 % en poids de 5,6-dihydroxyindoline, par rapport à l'agent total.

4. Procédé pour la nuance des cheveux, caractérisé en ce qu'on applique sur les cheveux une préparation de :
A) 0,1-5 % en poids d'une matière colorante naturelle et,
B) 0,1-5 % en poids d'un indoline de formule I selon la revendication 1,
dans un support aqueux sous forme d'émulsion ou de gel, on élimine par rinçage à l'eau l'agent tinctorial résiduaire et on sèche les cheveux à l'air.
